Europäisches Patentamt

European Patent Office

Office européen des brevets

(19)

(11) Publication number:

**0 392 556**

**A1**

(12) **EUROPEAN PATENT APPLICATION**

(21) Application number: **90107091.2**

(22) Date of filing: **12.04.90**

(51) Int. Cl.⁵: **C12P 19/12, C12P 19/18, C12N 9/10**

The microorganism(s) has (have) been deposited with FERM under number(s) BP-2838.

(30) Priority: **13.04.89 JP 91972/89**

(43) Date of publication of application:
**17.10.90 Bulletin 90/42**

(84) Designated Contracting States:
**DE DK GB IT NL**

(71) Applicant: **MEITO SANGYO KABUSHIKI KAISHA**
**41, 2-chome, Sasazuka-cho Nishi-ku**
**Nagoya-shi Aichi-ken(JP)**

(72) Inventor: **Higashi, Susumu**
**2-22-50, Noguchi-cho**
**Higashimurayama-shi, Tokyo(JP)**
Inventor: **Kuriiwa, Nobuo, Meito Sangyo**
**Kabushiki Kaisha**
**Hino-ryo, 6-10-4 Tamadaira**
**Hino-shi, Tokyo(JP)**
Inventor: **Iwasaki, Shinjiro**
**2-21-17, Higashitoyoda**
**Hino-shi, Tokyo(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Process for producing isomaltulose.**

(57) A process for producing isomaltulose, which comprises converting sucrose to isomaltulose in the presence of cells of a glucosyl transferase-producing microorganism of the genus Klebsiella or a treated product thereof is disclosed. And a process for producing isomaltulose, which comprises cultivating a glucosyl transferase-producing microorganism of the genus Klebsiella, and recovering isomaltulose from the culture medium is also disclosed.

EP 0 392 556 A1

This invention relates to a process for producing isomaltulose (also known as paratinose), and more specifically, to a process for producing isomaltulose in great quantities from sucrose efficiently by an enzyme reaction or by a fermentation method. The present invention also relates to a novel microorganism that can be used in the above process.

Known microorganisms having the ability to change carbohydrates to other carbohydrates, particularly those having the ability to convert sucroses to useful disaccharides include, for example, Leuconostoc mesenteroides, Protaminobacter rubrum, Serratia marcenscens, Serratia plymuthica, Erwinia carotovora, Erwinia carotovora var. atroseptica, Erwinia dissolvens, and Erwinia rhapontici. However, these isomaltulose-producing microorganisms yield glucose and fructose as by-products at the time of producing isomaltulose (6-O-α-D-glucopyranosyl-fructofuranoside). Accordingly, from the product, monosaccharides must be removed. This also affects the yield of isomaltulose from sucrose. Accordingly, the present inventors isolated many strains from the soil in order to search for a novel microorganism which does not produce monosaccharides as by-products. The inventors examined the ability of these organisms and also strains obtained from culture collections.

We found that certain strains of the genus Klebsiella, such as Klebsiella terrigena JCM 1687 [JAPAN COLLECTION OF MICROORGANISMS SECTION RIKEN (The Institute of Physical and Chemical Research)], and Klebsiella sp. No. 88 have the excellent ability to produce glucosyl transferase producing ability and are useful for converting sucrose to isomaltulose. It was found particularly that the Klebsiella sp. No. 88 strain has the excellent carbohydrate converting ability without the production of monosaccharides as by-products.

Thus, according to one aspect of this invention, there is provided a process for producing isomaltulose, which comprises converting sucrose to isomaltulose in the presence of cells of a glucosyl transferase-producing microorganism of the genus Klebsiella, or a treated product thereof.

According to another aspect of this invention, there is provided a process for producing isomaltulose, which comprises cultivating a glycosyl transferase-producing microorganism of the geuus Klebsiella in a culture medium containing sucrose, and recording the isomaltulose from the medium.

The present invention is characterized in that by utilizing glucosyl transferase, an enzyme produced by a microorganism of the genus Klebsiella, sucrose is converted to isomaltulose by an enzyme reaction or by a fermentation method.

Specific examples of the glucosyl transferase-producing microorganism of the genus Klebsiella which can be used in the process of this invention are Klebsiella terrigena JCM 1687 [deposited at RIKEN (Institute of Physical and Chemical Research)] at 2-1, Hirosawa, Wako-shi, Saitama-ken, Japan; freely available from the above Institution, and Klebsiella sp. No. 88 (deposited as FERM P-10640; transferred to an international deposition under the Budapest Treaty on March 30, 1990 under No. FERM BP-2838). The present invention, however, is not limited to these specific species. Any one skilled in the art can easily obtain glucosyl transferase-producing microorganisms of the genus Klebsiella that can be used in this invention by, for example, coating a soil suspension on an agar plate medium containing sucrose, cultivating it at 30 °C for 24 to 48 hours and separating the sucrose-utilizing microorgamism grown on the plate medium, cultivating the separated microorganism in a liquid medium containing sucrose at 30 °C for 72 hours under shaking, and determining the formation of isomaltulose by paper chromatography.

The Klebsiella sp. No. 88 that can be used in this invention is a novel microorganism not described in the prior literature and has the following bacteriological properties.

The identification of the above microorganism was performed mainly on the basis of Manual of Methods for General Bacteriology (edited by U. S. Society of Microbacteriology, 1981), and "Taxonomy and Identification of Microorganisms" (edited by Takeji Hasegawa, T.odai Shuppankai, 1985).

1. Morphology

Agar broth, cultivated at 37 °C: Rods usually 1.0 to 1.5 x 2.0 to 4.0 micrometers. Singly or in a straight chain-like pair. Rarely, chain cells were observed. No morphism nor motility. No spore. No anti-acid. Gram-negative. Presence of a pellicle was noted.

II. Cultural properties

(1) Cultivation on broth-agar plate at 37 °C
Shape: Circular, grew to a size of 2 mm in 24 hours.

Peripheral edge: entire

Protrusion: flat or semi-lenticular

Gloss: glossy

Surface: smooth

Color: non-transparent: homogeneous butter-like, white.

     (2) Broth-agar slant culture, 37 °C

Growth: good

Shape: filamentous

     (3) Groth liquid culture: 37 °C

Growth: good, entirely grew, and turbid.

Sedimentation: little

Coloration and decoloration: none

     (4) Stab culture on broth-gelatin

Grew at 15 °C along the stab line. Does not liquefy gelatin.

Does not liquefy gelatin at 37 °C.

     (5) Litmus milk, 37 °C

Litmus fading. Coagulated. No liquefaction. Acidified.

III. Physiological properties .

     (1) Reduction of nitrate: positive

     (2) Denitration reaction: positive

     (3) MR test: negative

     (4) VP test: positive

     (5) Formation of indole: negative

     (6) Formation of hydrogen sulfide: negative

     (7) Hydrolysis of starch: negative

     (8) Utilization of citric acid: positive in the Koser medium, Christensen medium and Simmons medium.

     (9) Utilization of inorganic nitrogen sources: Both ammonium salts and nitrate salts can be utilized.

     (10) Formation of pigment: none

     (11) Urease: positive

     (12) Oxidase: negative

     (13) Catalase: positive

     (14) Growth range: pH 6 to 10

Temperature: 10-44.5 °C

     (15) Behavior to oxygen: facultatively anaerobic

     (16) O-F test (Hugh-Leifson) Ferments D-glucose

     (17) Formation of acids and gases from carbon sources

|  | Acid | Gas |
|---|---|---|
| L-arabinose | + | + |
| D-xylose | + | + |
| D-glucose | + | + |
| D-mannose | + | + |
| D-fructose | + | + |
| D-galactose | + | + |
| Maltose | + | + |
| Sucrose | + | + |
| Lactose | + | + |
| Trehalose | + | + |
| D-sorbitol | + | + |
| D-Mannitol | + | + |
| Inositol | + | + |
| Glycerol | + | + |
| Starch | + | + |
| Adonitol | - | - |
| Dulcitol | - | - |
| Esculin | + | + |
| Salicin | + | + |
| Inulin | - | - |
| D-melezitose | - | - |
| Sorbose | + | + |
| L-rhamnose | + | + |
| Raffinose | + | + |
| d-Tartaric acid | + | + |

(18) Oxidation of gluconic acid (by the iron gluconate citrate medium of Korth et al.): negative

(19) Utilization of malonic acid: positive

(20) Utilization of organic acids (by the Kauffmann-Petersen medium)

Citrate salt (sodium: positive)

d-tartrate: positive

Mucic acid: positive

(21) Utilization of carbon compounds

Gentisic acid: negative

m-Hydroxybenzoic acid: negative

(22) Utilization of organic acid nitrogen sources:

Sodium L-glutamate: positive

L-hydroxyproline: positive

(23) Decomposition of arginine (by the Møller method): negative

(24) Decarboxylation of lysine (by the Møller method): positive

(25) Decarboxylation reaction of ornithine (by the Møller method): negative

(26) Decarboxylation of glutamic acid (by the Møller method): negative

(27) Deamination reaction of phenylalanine: negative

(28) Liquefaction of pectinic acid (by the method of Starr): negative

(29) Fecal coliform test (formation of a gas from lacose at 44.5 °C): negative

IV. G-C ratio of DNA: 58.0 %

The above microorganism strain was examined for the identity of difference with or from known microorganism strains on the basis of Bergey's Manual of Determinative Bacteriology, 8th edition (1974) and Manual of Systematic Bacteriology, 9th edition (1984). As a result, the microorganism strain discovered by the present inventors showed the closest properties to those of JCM 7251 (ATCC 33531), a standard strain of Krebsiella planticola. However, the present microorganisms showed differences in respect of the methyl red test, and the fermentation and utilization of adnitol, starch, and d-tartaric acid. Since a microorganism strain of the genus Krebsiella capable of producing isomaltulose has not been found in the literature, the present strain is considered to be a novel microorganism strain characterized by producing isomaltulose.

Accordingly, the present inventors named the above microorganism strain as Klebsiella sp. No. 88 and deposited it as FERM P-10604 at Patent Microorganism Deposition Center of Fermentation Research Institute at 1-1-3, Higashi, Tsukuba-shi, Ibaraki-ken, Japan, which was transferred to an international deposition under the Budapest Treaty on March, 30, 1990 and given FERM BP-2838.

In the present invention, strains belonging to the same genus as above and having the ability to convert carbohydrates, and mutants of these may also be used in addition to the above strain.

The microorganisms used in the process of this invention can be cultivated suitably by a known method in a culture medium containing sucrose as a main carbon source. The medium may be a synthetic or natural medium. Examples of the nitrogen sources that can be included in the medium are inorganic nitrogen compounds such as nitrate salts and ammonium salts and organic nitrogen-containing compounds such as urea, corn steep liquor, a hydrolyzate of casein, peptone, yeast extract, meat extract and amino acids. Examples of inorganic salts include, for example, potassium salts and sodium salts which may be added in small amounts.

The concentration of the sucrose in the medium may be 2 to 40 % by weight. From the standpoint of growth and proliferation of the cells, it is desirably 2 to 30 % by weight, preferably 2 to 20 % by weight.

The cultivation may be carried out at room temperature to about 40 °C, preferably about 30 to about 37 °C under neutrality to weak acidity, especially at a pH of 5 to 7, facultatively anaerobically or aerobically. The method of cultivation may be batchwise or semi-batchwise.

The cultivation time differs depending upon the concentration of sucrose in the medium, but a period of about 16 to 48 hours is suitable. After the cultivation, the cells are separated from the culture. The separation may be effected by known methods, for example, by centrifugal separation or filtration. When the cells are to be separated by a centrifugal method, various flocculat ing agents may be added to the culture so that the cells may be flocculated and separated easily and efficiently. The supernatant culture resulting after removal of the cells is subjected to heating, filtration and ion-exchange resin treatment, and then evaporated and concentrated to a solids content of, for example, 70 to 80 % by weight. The concentrate is then gradually cooled to about 20 °C with slow stirring to precipitate crystals of isomaltulose. The resulting crystals can be separated from the mother liquor by subjecting it to a basket-shaped centrifugal separator. The molasses may be sent to a recrystallization step as required, and can be re-utilized.

The cells separated from the culture contains glucosyl transferase, and can be used in an enzyme reaction of converting to isomaltulose. The cells or a treated product thereof may be used directly or after the cells is included in a fixing carrier. Or as required, it may be used after it is subjected to crosslinking treatment with a crosslinking agent. It is particularly desirable to use the cells or the treated product after fixation on a suitable carrier. The fixation may be carried out by known methods. For example, the fixation may be performed by including the cells or the treated product thereof in an acrylamide-type monomer, and then polymerizing it, or by a method which comprises including the cellls or the treated product thereof in a carrier, and then treating the product with a crosslinking agent, for example, by the chitosan-glutaraldehyde method, the carrageenin-glutaraldehyde method, or the alginic acid-glutaraldehyde method.

The "cells or the treated product thereof", as used in this invention, means not only the cells themselves; the so-called "crude enzyme" obtained by disrupting the cells by ultrasonication or by immersing them in a surface-active agent such as sodium dodecylsulfate, or removing the cell membranes from the product, or "purified enzymes" obtained by separating enzyme active fractions in various purities.

The converting reaction of sucrose using the above-mentiond cells of the treated product thereof which is optionally fixed may be carried out by adding the cells of the treated product thereof to an aqueous medium usually containing sucrose in a concentration of 10 to 75 % by weight, preferably 30 to 50 % by weight, more preferably 30 to 40 % by weight, and incubating it at a temperature of about 25 to about 40 °C, preferably about 30 to atout 37 °C for about 20 to about 40 hours. At this time, the pH of the reaction solution need not to be particularly controlled.

The resulting isomaltulose may be separated from the reaction solution and/or purified by known methods such as desalting, decoloration, concentration, crystallization or separated sugar.

The following Examples illustrate the invention more specifically.

Example 1

Klebsiella sp. No. 88 (FERM BP-2838) was inoculated in a 200 ml Erlenmayer flask containing 100 ml of a culture medium adjusted to a pH of 6.0 and containing 20 g/dl of sugar, 0.16 g/dl of yeast extract and 0.43 g/dl of $K_2HPO_4$, and subjected to stationary culture at 30 °C for 24 hours. All the cultivated product was inoculated in 1 liter of a culture medium having the same composition, and subjected to stationary culture at the same temperature for 24 hours.

After the cultivation, the cells were removed by a centrifugal separator, and the supernatant was recovered. The sugar composition of the supernatant was measured by HPLC, and was found to be 85 % of isomaltulose and 15 % of trehalulose (1-O-α-D-glucopyranosyl-fructofuranose). No other sugar was detected.

The supernatant was passed through an ion-exchange resin 1R 120B and 1RA-411 (a product of Organo Co. Ltd.) for desalting. It was then decolorized with active carbon, and concentrated to a sugar concentration of 75 % by weight to precipitate isomaltulose. The recovery rate of the crystals was 70 % based on sucrose.

Example 2

Klebsiella terrigena JCM 1687 was inoculated in a 500 ml shouldered flask containing 50 ml of the same culture medium as described in Example 1, and subjected to shaking culture at 37 °C for 24 hours. 5 ml of the cultivation product was inoculated in 50 ml of a culture medium having the same composition and cultivated under shaking for 48 hours. The cells were removed by a centrifugal separator from the liquid obtained after the cultivation, and the supernatant was recovered. The sugar composition of the supernatant was examined by TLC, and the presence of about 10 % of isomaltulose and unreacted sucrose was determined.

Example 3

Klebsiella sp. No. 88 was inoculated in a 200 ml Erlenmeyer flask containing 100 ml of a culture medium adjusted to a pH of 6.0 and containing 20 g/dl of sugar, 0.48 g/dl of corn steep liquor and 0.2 g/dl of $KH_2PO_4$, and subjected to stationary culture at 30 °C for 24 hours. Sucrose (30 g) was added to the cultivation product, and the mixture was further cultivated at 30 °C for 24 hours. Subsequently, the same step as described in Example 1 was carried out. As a result, crystalline isomaltulose was recovered in a ratio of 75 % based on sucrose.

Example 4

Klebsiella sp. No. 88 was cultivated in the same culture medium as described in Example 1 to give 100 ml of a cell slurry.

Equal weights of the cell slurry and 4 % carrageenin were mixed, and added dropwise to a 3 % KCl solution to include the cellls. The included cells were further crosslinked with polyethylenimine and glutaraldehyde solution to fix the cells. 200 ml of the fixed cells were filled into a column having a volume of 300 ml. 40 % sucrose solution was passed through the column at an SV of 0.2 to 0.3 at a temperature of 30 to 37 °C. The sucrose solution was converted to 85 % of isomaltulose and 15 % of trehalulose (1-O-α-D-glucopyranosyl-fructofuranose).

**Claims**

1. A process for producing isomaltulose, which comprises converting sucrose to isomaltulose in the presence of cells of a glucosyl transferase-producing microorganism of the genus Klebsiella or a treated product thereof.

2. The process of claim 1 in which the microorganism is Klebsiella terrigena JCM 1687 or Klebsiella sp. No. 88 (FERM BP-2838).

3. The process of claim 1 in which the cells or the treated product thereof is fixed to a carrier.

4. The process of claim 1 in which the conversion is performed by using an aqueous medium containing sucrose in a concentration of 10 to 75 % by weight.

5. The process of claim 1 in which the conversion is carried out at a temperature of about 25 to about 40 °C.

6. A process for producing isomaltulose, which comprises cultivating a glucosyl transferase-producing microorganism of the genus Klebsiella, and recovering isomaltulose from the culture medium.

7. The process of claim 6 in which the microorganism is Klebsiella terrigena JCM 1687 or Klebsiella sp. No. 88 (FERM BP-2838).

8. The process of claim 6 in which the culture medium contains sucrose in a concentration of 2 to 50 % by weight.

9. The process of claim 6 in which the cultivation is carried out at a pH of 5 to 7.

10. Klebsiella sp. No. 88 strain (FERM BP-2838).

| | DOCUMENTS CONSIDERED TO BE RELEVANT | | EP 90107091.2 |
|---|---|---|---|

| Category | Citation of document with indication, where appropriate, of relevant passages | Relevant to claim | CLASSIFICATION OF THE APPLICATION (Int Cl⁵) |
|---|---|---|---|
| A | <u>DE - A1 - 3 528 752</u> <br> (BAYER AG) <br> * Claims; example 1; especially page 20, lines 24-31 * <br> -- | 1-10 | C 12 P 19/12 <br> C 12 P 19/18 <br> C 12 N 9/10 |
| A | <u>DE - A1 - 3 038 219</u> <br> (SÜDDEUTSCHE ZUCKER AG) <br> * Claims; page 12, lines 13-16 * <br> -- | 1-5 | |
| A | <u>GB - A - 2 063 268</u> <br> (TATE & LYLE LTD.) <br> * Claims * <br> -- | 1-5 | |
| A | <u>EP - A2 - 0 091 063</u> <br> (SÜDDEUTSCHE ZUCKER AG) <br> * Claims * <br> -- | 1-5 | |
| A | <u>EP - A1 - 0 028 900</u> <br> (TALERS DEVELOPMENT N.V.) <br> * Claims * <br> -- | 1-5 | TECHNICAL FIELDS SEARCHED (Int Cl⁵) |
| A | <u>EP - A1 - 0 001 099</u> <br> (BAYER AG) <br> * Claims * <br> ---- | 1-10 | C 12 P 19/00 |

The present search report has been drawn up for all claims

| Place of search | Date of completion of the search | Examiner |
|---|---|---|
| VIENNA | 16-07-1990 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82